(19) Europäisches Patentamt
European Patent Office
Office européen des brevets

(11) Publication number : **0 665 293 A2**

# (12) EUROPEAN PATENT APPLICATION

(21) Application number : **95300468.6**

(51) Int. Cl.$^6$ : **C12Q 1/68**

(22) Date of filing : **26.01.95**

The applicant has subsequently filed a sequence listing and declared, that it includes no new matter.

(30) Priority : **26.01.94 JP 6971/94**

(43) Date of publication of application : **02.08.95 Bulletin 95/31**

(84) Designated Contracting States : **CH DE FR GB LI**

(71) Applicant : **HAMAMATSU PHOTONICS K.K.**
**1126-1 Ichino-cho**
**Hamamatsu-shi**
**Shizuoka-ken (JP)**

(72) Inventor : **Hosoi, Shigeru**
**c/o Hamamatsu Photonics K.K.,**
**1126-1, Ichino-cho**
**Hamamatsu-shi, Shizuoka-ken (JP)**
Inventor : **Fukami, Tadashi**
**c/o Hamamatsu Photonics K.K.,**
**1126-1, Ichino-cho**
**Hamamatsu-shi, Shizuoka-ken (JP)**
Inventor : **Hiyoshi, Makiko**
**c/o Hamamatsu Photonics K.K.,**
**1126-1, Ichino-cho**
**Hamamatsu-shi, Shizuoka-ken (JP)**

(74) Representative : **West, Alan Harry et al**
**R.G.C. Jenkins & Co.**
**26 Caxton Street**
**London SW1H 0RJ (GB)**

## (54) Method of determining base sequence of nucleic acid.

(57) A method of determining the base sequence of single-stranded nucleic acid, including : a first step of hybridizing each of plural primers comprising oligonucleotide, with a template comprising a single-stranded nucleic acid to be examined, thereby to form a complex having a double-stranded portion comprising a portion of the template and the primer, the plural primers being separately disposed in a plurality of regions in accordance with the kinds of the primers ; a second step of substantially simultaneously adding plural kinds of nucleotides or nucleotide analogues to the complexes respectively disposed in the plural regions, thereby to perform growth reaction of the primer with the nucleic acid to be examined as the template in a direction of from 5' to 3' of the primer, the plural kinds of nucleotides or nucleotide analogues being complementary to nucleotides constituting the template in the complex, and being capable of forming base pair with nucleotides constituting the template ; and a third step of detecting the amount of the growth of the primers.

EP 0 665 293 A2

The present invention relates to a method of determining the base sequence of a nucleic acid, which is capable of providing information on a gene of an organism by analyzing nucleic acid sequences contained in the organism by use of a physical and/or chemical technique.

As the method of determining the base sequence of a nucleic acid, two techniques, i.e., the dideoxy chain termination method developed by Sanger et al. (Sanger, F., Nicklen, S. and Coulson, A. R.; Proc. Natl. Acad. Sci. USA 74: 5463-5467, 1977), and the chemical sequencing method developed by Maxam-Gilbert (Maxam, A. M. and Gilbert, W.; Proc. Natl. Acad. Sci. USA 74: 560-564, 1977), have been put to practical use. In either of these methods, fragments of DNA (deoxyribonucleic acid) to be examined are provided by using a synthetic or decomposing technique so that the resultant fragments may be labelled with a radioisotope, a fluorescent dye, etc., for the purpose of identifying the kinds of the terminal base of the fragments having various lengths; then the resultant product is subjected to gel electrophoresis so as to separate the fragments from each other in an order of from the shortest fragment toward longer fragments in accordance with the length thereof; and the kind of the terminal base of the fragment is read out in an order of from the shortest fragment toward longer fragments, whereby the sequence of the above DNA to be examined is estimated. These methods have been improved in various manners up to the present. However, either of these methods employs a separation technique based on electrophoresis, and therefore they require a great deal of time and efforts. Accordingly, for the purpose of determining a large-scale base sequence such as the base sequence of the entire human genome (base sequence comprising about three billion base pairs), a new method capable of achieving a better efficiency has been strongly desired.

As one of such "new methods", a method called as "sequencing by hybridization (hereinafter, referred to as "SBH") method" has been proposed (Bains, W and Smith, G. C., J. Theor. Biol. 135: 333-307, 1988; Darmance, R., Labat, I., Brukner, I. and Crkvenjakov, R., Genomics 4: 114-128, 1989).

This method basically has a principle such that the sequence of a DNA clone to be examined is determined by sequencing (or arranging) a composition comprising $F^K$ of all oligonucleotides having a k-base length (i.e., a length of oligonucleotide corresponding to k-bases or k-base pairs) constituting the DNA to be examined. The algorithm for the above sequencing may be attributed or reduced to a problem of single-stroke picturing of a figure or diagram, which often arises in a puzzle (Pevzner, P. A., J. Biomol. Struct. Dyn. 7: 63-73, 1989). In 1991 Pevzner et al. have determined by simulation the length of a DNA clone, which may be determined with a probability of 95 % on the basis of the composition of oligonucleotides having a length of k-base pairs (Pevzner, P. A., Lysov, Y. P., Khrapko, K. R. Belyavskii, A. V., Florent ev, V. L . and Mirzabekov, A., J. Biomol. Struct. Dyn. 9: 399-419, 1991). In this method, for example, when oligonucleotides having an 8-base length in 65536 combinations are used, the base sequence of a DNA having a length of about 180 bases may be determined, as shown in Figs. 12A and 12B. In the actual sequencing method, a single strand of nucleic acid to be examined is subjected to hybridization(crossing) together with oligonucleotides having length of k bases, thereby to find the resultant composition of $F^K$. Accordingly, when the above oligonucleotides have the length of 8-bases, a nucleic acid clone to be examined having a length exceeding about 180 bases cannot be used for the hybridization with the above oligonucleotides. Furthermore, in this case, since 65536 combinations of oligonucleotides are used, in view of the constitution of a practically usable apparatus, it is preferred to fix the oligonucleotides or the nucleic acid to be examined to a matrix-like member having a size such that it may be set or placed in a measuring apparatus. Such a method is called as "sequencing by hybridization with oligonucleotide matrix hereinafter, referred to as "SHOM") method.

However, it has been pointed out that SBH and SHOM as described above have two serious problems (Akira Suyama, "Protein, Nucleic Acid and Enzyme" (in Japanese), 38: 647-657, 1993). One of these two problems is the problem of mismatch due to incomplete hybridization or partial hybridization. When one of the oligonucleotides is not completely complementary to the nucleic acid to be examined but is partially complementary to the nucleic acid to a certain extent, the oligonucleotide can still be crossed or hybridized with the nucleic acid to be examined. In such a case, it is possible that a certain sequence, which is not originally included in the nucleic acid to be examined, is included or involved in the calculation for the sequencing or arranging. Further, the other of the above two problems is the problem of repetitive sequences (or the identical nucleotide sequences). If a plurality of the same nucleotide sequences are included in a nucleic acid clone to be examined, it is possible to recognize the inclusion of the above nucleotide (base) sequences in the nucleic acid to be examined by detecting the crossing therebetween. In such a case however, it is extremely difficult to determine the respective positions of the repetitive base sequences only by utilizing the arrangement based on a computing (or arithmetic) algorithm.

In addition, there is a case where the SBH method cannot univocally determine a base sequence to be detected even when the nucleic acid to be examined does not include repetitive repeat sequences. Hereinbelow, a simple example of such a case is described.

First, it is supposed that a nucleic acid clone to be examined has a sequence of [ATGAT]. In this case,

when a primer comprising a trimer (3-mer) is hybridized with the above nucleic acid to be examined, partial sequences (corresponding to a trimer) in the nucleic acid to be examined are [ATG], [TGA], and [GAT]. However, when the original sequence of the nucleic acid to be examined is intended to be determined by arranging these three kinds of partial sequences, the following two cases may be considered with respect to the base sequence of the nucleic acid to be examined:

(Case 1)

```
ATG
   TGA
      GAT,
```

and,

(Case 2)

```
GAT
   ATG
      TGA.
```

Accordingly, in such a case, the SBH method cannot univocally determine the base sequence of the nucleic acid to be examined.

The SBH or SHOM method as described above has markedly shortened the period of time required for the operation or treatment therefor, as compared with that in the sequencing methods prior to the SBH or SHOM method. However, for the purpose of analyzing an actual gene, it has been desired to determine the longer base sequence of a nucleic acid clone to be examined in a shorter period of time than that for the above SBH or SHOM method.

An object of the present invention is to provide a method of determining the base sequence of nucleic acid, which has solved the above problems encountered in the prior art.

Another object of the present invention is to provide a method of determining the base sequence of nucleic acid, which is capable of rapidly determining the base sequence of the nucleic acid to be examined.

According to the present invention, there is provided a method of determining the base sequence of a single-stranded nucleic acid molecule, comprising:

a first step of hybridizing each of the plural primers comprising all possible fixed-length oligonucleotides, with a template comprising a single-stranded nucleic acid to be examined, thereby to form a complex having a double-stranded portion comprising a portion of the template and the primer; the plural primers being separately disposed in a plurality of regions in accordance with the kinds of the primers;

a second step of adding plural kinds of nucleotides or nucleotide analogues to the complexes respectively disposed in the plural regions, thereby to perform a growth reaction of the primer with the nucleic acid to be examined as the template in a direction of from 5' to 3' of the primer; the plural kinds of nucleotides or nucleotide analogues being complementary to the nucleotide constituting the template in the complex, and being capable of forming base pair with the nucleotide constituting the template; and

a third step of detecting the amount of the growth of the primer.

According to the above-described base sequence-determining method of the present invention, in the first step, nucleic acid to be examined is hybridized with primers which have been separately disposed in a plurality of regions in accordance with the kinds of the primers, thereby to form complexes thereof; and in the second step, the plurality of nucleotides or nucleotide analogues, which are capable of matching with the nucleic acid to be examined as a template, are substantially simultaneously added to the above complexes thereby to cause the growth (or extension) reaction in the direction of from 5' to 3' of the primer at one operation, in accordance with the nucleic acid to be examined as the template. Then, in the third step, the amount or degree of the growth corresponding to each of the primers. Through such a procedure, the amount of each growth based on each of the primers disposed in each of the above plural regions, i.e., the position of the binding of each primer with the nucleic acid to be examined, may be recognized. In addition, since the kinds of the primers disposed in the respective regions are different from each other, the base sequence of the nucleic acid to be examined may be determined by analyzing the base sequences of the primers by utilizing an algorithm for so-called "single-stroke picturing".

Further, in a case where the above-mentioned "plural regions" are defined or provided by respective columns formed in a so-called "capillary plate" (such as one comprising a glass material or a silicon material), the columns are disposed separately and independently from each other, and therefore the primers disposed in different regions (i.e., different columns in the capillary plate) are not mixed with each other. The "capillary plate" used herein refers to a plate-like member having a plurality of through holes (columns) which have been formed in the plate-like member and extend in the thickness direction of the plate-like member. Each of the above columns may preferably have a diameter of about 1-500 μm (more preferably, about 6-30 μm). The plate-like member may preferably have a thickness (i.e., the length of the columns) of about 0.1-3.0 mm (more preferably, 0.5-1.0 mm). The plate-like member may preferably have about 10,000-10,000,000 columns (more preferably, about 65,000-1,000,000 columns) with respect to a predetermined area (having a size of 2.5 cm × 2.5 cm or smaller) of the surface of the plate-like member. Such a capillary plate may for example have a plate-like structure comprising a large number of glass pipes having an inner diameter of about 6-30 μm which have been formed into a bundle-like shape (with respect to the details of the capillary plate, e.g., a technical paper entitled "Principle and Application of Capillary Plate" (in Japanese), published by Hamamatsu Photonics K.K. (June 1992) may be referred to).

In view of easiness in the sequencing of nucleic acid to be examined, it is preferred to use a capillary plate having a square shape, and having columns arranged in a matrix-like form. However, the shape of the capillary plate usable in the present invention is not particularly restricted. More specifically, a capillary plate having a circular shape, a triangular shape, etc., may also be used in the present invention.

In a case where the columns of the capillary plate are defined by cylindrical holes having a diameter of a few microns to a few hundred of microns, when a liquid such as one comprising a primer or nucleic acid to be examined is introduced into the each column, it is preferred to utilize electrophoresis to be conducted in a direction corresponding to the thickness direction of the capillary plate, in view of the surface tension of the liquid. In addition, it is preferred to fix each of the primers into each of the columns formed in the capillary plate, in view of easiness in the sequencing of the nucleic acid to be examined.

As the method for detecting the above amount of the growth, various methods may be used without particular limitation. For example, the amount of the growth based on primer pairing (hereinafter, referred to as "primer growth") in the third step may also be determined in a manner such that pyrophosphoric acid, which has been released from the plural kinds of nucleotides or nucleotide analogues along with the above growth reaction in the second step, is caused to react with APS (adenosine 5'-phosphosulfate) to produce ATP (adenosine 5'-triphosphate), and the quantity (amount) of light emitted on the basis of the reaction of the ATP with luciferase is detected or measured. In such a case, the quantity of light emitted on the basis of the reaction of ATP is proportional to the amount of the primer growth, and therefore the length of the grown primer and the position or site of the binding of the primer with the nucleic acid to be examined may be determined.

Alternatively, it is also possible to utilize the following method. In the second step described above, plural kinds of deoxyribonucleotide triphosphates (dNTP) labelled with a fluorescent substance are added as the above plural kinds of nucleotide analogues to the reaction system, and the growth reaction of the primer with the nucleic acid to be examined as a template is caused. Then, the above-mentioned plural regions are irradiated with excitation light, and the quantity of light emitted from the fluorescent label of the thus grown primer is detected or measured to determine the amount of the primer growth in the third step. Through the above procedure, since the quantity of light emission observed from each of the complexes based on irradiation with excitation light is proportional to the amount of the growth, the length of the growth and the position of the binding of the primer with the nucleic acid to be examined may be determined.

Further scope of applicability of the present invention will become apparent from the detailed description given hereinafter. However, it should be understood that the detailed description and the specific examples, while indicating preferred embodiments of the invention, are given by way of illustration only, since various changes and modifications within the spirit and scope of the invention will become apparent to those skilled in the art from this detailed description.

Fig. 1 is a schematic perspective view showing a capillary plate to be used in an embodiment of the present invention.

Fig. 2 is a diagram for illustrating the arrangement of plural primers to be disposed in the capillary plate as shown in Fig. 1.

Fig. 3 is a schematic sectional (partially perspective) view for illustrating the procedure to be conducted in First Embodiment of the present invention appearing hereinbelow.

Figs. 4A to 4F are illustrating the procedure to be conducted in First Embodiment of the present invention appearing hereinbelow.

Fig. 5 is a schematic perspective view showing the structure of an apparatus for determining a base sequence used in the First Embodiment.

Fig. 6A is showing the emission of light after primer growth.

Fig. 6B is illustrating the procedure to identify a base sequence of a nucleic acid to be examined to be conducted in the capillary plate as shown in Fig. 1.

Fig. 7 is a schematic view for illustrating partial identification of a long-strand (or long-chain) nucleic acid to be examined.

Fig. 8 is a schematic sectional (partially perspective) view for illustrating the procedure to be conducted in Second Embodiment of the present invention appearing hereinbelow.

Figs. 9A and 9B are illustrating the procedure to be conducted in Second Embodiment of the present invention appearing hereinbelow.

Fig. 10A is a schematic perspective view showing the structure of an apparatus for determining a base sequence used in Second Embodiment.

Fig. 10B is showing fluorescence selectively transmitted having a wavelength longer than the excitation light wavelength.

Fig. 10C is showing excitation light selectively transmitted.

Fig. 10D is showing an ultra-high pressure mercury lump as an ecitation light source.

Fig. 11 is an experimental result showing the relationship between wavelength and the intensity of light emitted from DNA labelled with a fluorescent substance measured in the Second Embodiment. A:fluorescein, B:rhodamine.

Fig. 12A is a schematic view for illustrating the conventional SBH method.

Fig. 12B is illustrating a procedure to determine the base sequence for a nucleic acid.

First Embodiment

(1) First, referring to Fig. 1, a glass capillary plate (CA) in a square shape having plural columns formed therein is provided, and a plurality of oligonucleotides including all of the combinations comprising k bases as plural primers are fixed into the above respective columns, in the same manner as in the SBH or SHOM method as described above. In Fig. 1, trimers (3 mers) of all of the combinations containing A, T, G, and C, are fixed into 64 columns of the glass capillary plate.

(a) These oligomers may be prepared by means of an automatic DNA synthesizing apparatus such as a commercially available apparatus (trade name: 381A) mfd. by Applied Biosystems Inc.

(b) The primers thus synthesized may be fixed into the capillary plate in the following manner.

1. An amino group ($-NH_2$) is fixed to the inside of each of the above columns. In this fixing, portions of the capillary plate CA other than the above columns are firstly masked, and the thus masked capillary plate CA is placed in a closed or hermetic vessel such as desiccator. Then, aminopropyl trimethoxysilane is evaporated and the closed vessel is filled with the resultant gas, and the vessel is left standing for several hours. Successively, the resultant capillary plate CA is subjected to baking in an oven (baking furnace) at about 160 °C.

2. Then, then amino group ($-NH_2$) is bound to the 5'-end of the primer synthesized by an automatic synthesizing apparatus. For example, a group of $NH-(CH)_2-NH_2$ is fixed to the 5'-end by causing the primer to react with $H_2N(CH_2)_2NH_2$ and carbodiimide (at pH6).

More specifically, this reaction may be represented by the following reaction formula or equation.

[Formula 1]

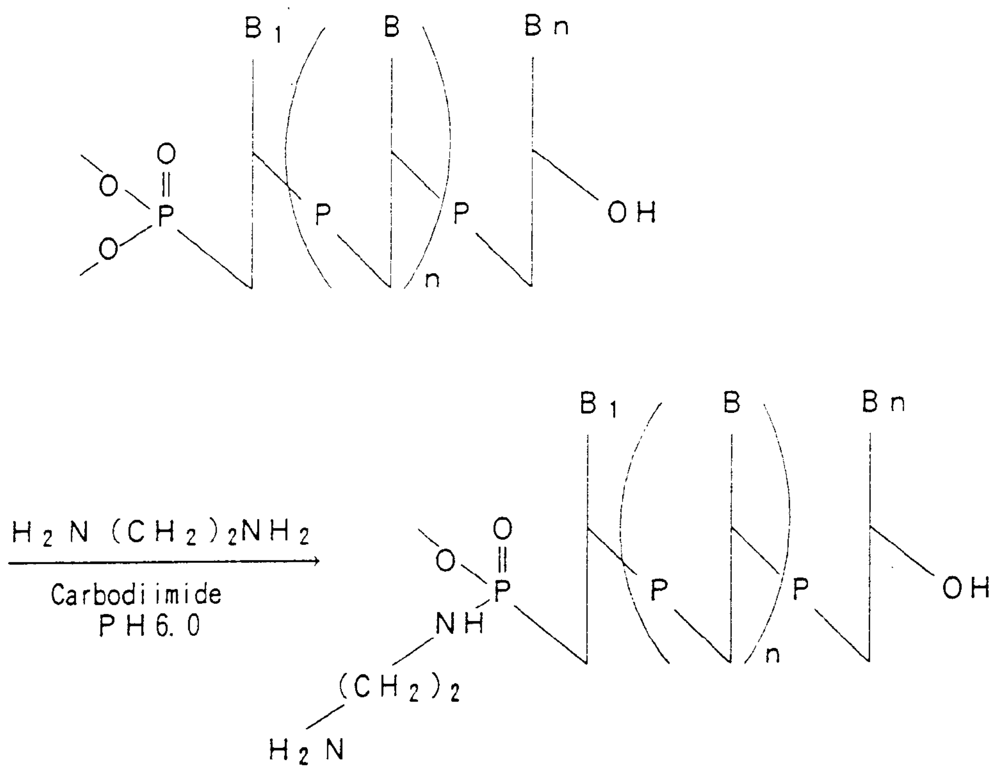


In the above Formula 1, "$B_a$" denotes an n-th base group selected from A (adenine), T (thymine), G (guanine) and C (cytosine).

3. Next, a bifunctional crosslinker (or crosslinking agent) which is capable of forming a covalent bond with the amino group ($-NH_2$), such as disuccinimidyl suberate represented by the following Formula 2, or a water-soluble homologue thereof, i.e., bis-sulfusuccinimidyl suberate represented by the following Formula 3, is added to the above reaction system, thereby to produce the product represented by the following Formula 4.

[Formula 2]

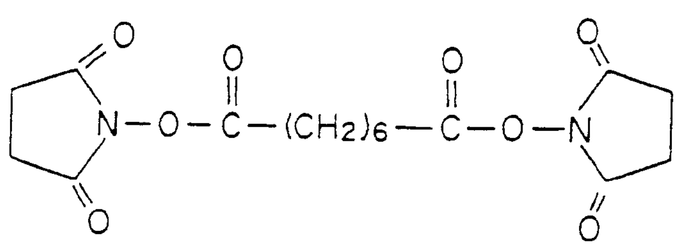

[Formula 3]

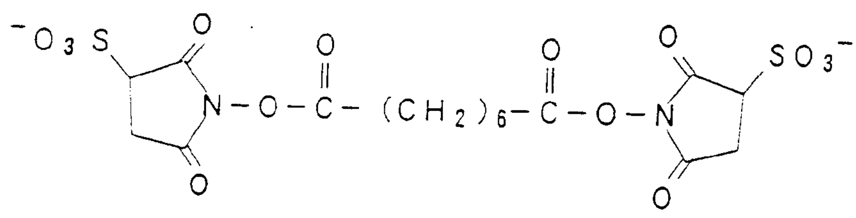

[Formula 4]

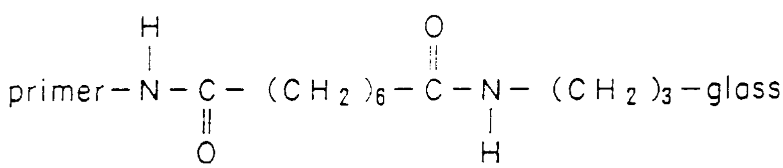

When the above reaction is utilized, it is possible that the plural primers themselves are bound to each other. In such a case, it is preferred that a COOH group is added to the aminopropyl trimethoxysilane, or a COOH group is added or introduced into the amino group which has been fixed to the column (i.e., the surface of the glass), so that the glass-COOH group is succinimidylated (or provided with a succinimidyl group) as described above.

At this time, when the above glass-COOH group is activated by use of N-hydroxysuccinimide represented by the following Formula 5, a product represented by the following Formula 6 is produced. When the thus obtained product is mixed with the aminated primer obtained in the above Step 2, these molecules are crosslinked to each other.

[Formula 5]

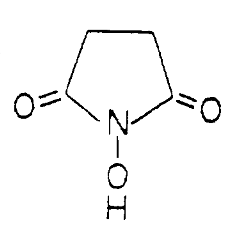

[Formula 6]

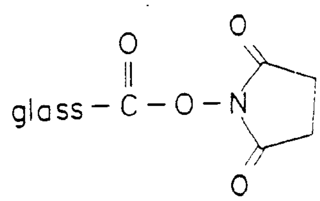

Further, when the bifunctional crosslinker and the aminated primer are added with a time lag between the addition thereof (i.e., when the aminated primer is first added to the glass-linker product and then the aminated primer is added thereto), a glass-(linker)-primer product may be obtained. In other words, the primer may be fixed to the glass with a high efficiency, as represented by the following Formula 7.

[Formula 7]

Glass-linker

↓

Glass-linker + aminated primer

↓

Glass-linker-primer

Moreover, a primer may be efficiently fixed to an -SH group which has been introduced into each of the columns, by use of a procedure represented by the following Steps 1-3.

1. First, mercaptopropyl trimethoxysilane is caused to react with the surfaces of all columns to fix an -SH group thereto. Such fixing may be effected by causing the above-described column-amino group to react with the "Traut reagent" represented by the following Formula 8, thereby to add an -SH group thereto.

[Formula 8]

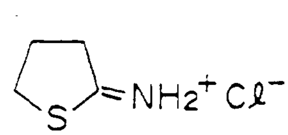

2. Next, an amino group is added in advance to the 5'-end of each primer in the same manner as described above, and then sulfosuccinimidyl-4-(N-maleimidomethyl) cyclohexane-1-carboxylate (sulfo-SMCC) as a bifunctional cross-linker is bound to the resultant product so as to form a covalent bond therebetween (at pH7). The thus obtained product is represented by the following Formula 9.

[Formula 9]

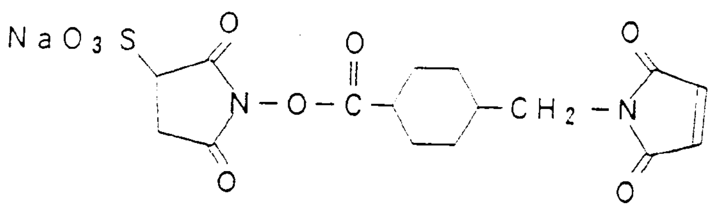

3. Each of the primers which has been activated in the above-mentioned manner reacts with the -SH group to form a covalent bond. Accordingly, in a case where different primers are separately introduced into the respective columns (at pH6), the activated primer is fixed to the column as shown by the following Formula 10.

[Formula 10]

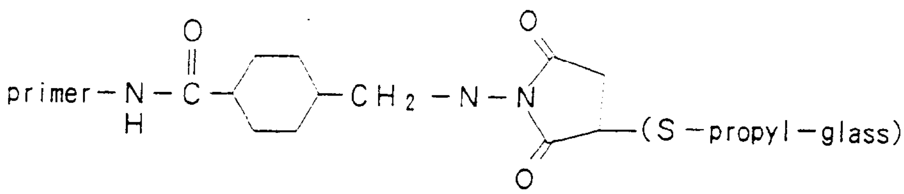

In the above-described columns of capillary plate CA shown in Fig. 1, the primers are arranged so as to provide oligonucleotide compositions as shown in Fig. 2. In view of surface tension of liquid (comprising the primer) to be injected into the column, each primer may preferably be injected into the column by inserting a primer injection needle connected to the automatic synthesizing apparatus into the column, and applying an electric field in the direction of thickness of the capillary plate CA to conduct electrophoresis. As a result of the above procedure, each of the oligonucleotides is fixed into each column as shown in Fig. 1, in which a portion of the capillary plate CA is hypothetically taken out therefrom and enlarged to schematically show the resultant state. In Fig. 1, the primer has a sequence of [CCC].

(2) Then, into one of the columns in the capillary plate CA having a primer fixed thereto (for example, into the column having the primer with the sequence of [GAC] as shown in Fig. 3), unknown single-stranded nucleic acid to be examined (for example, one having a base sequence of [AGCTGCTA]) is introduced (Fig. 3). Based on such introduction, the primer disposed in each column is hybridized (or crossed) with the nucleic acid to be examined. Then, the nucleic acid to be examined which has not been hybridized with the primer is washed out. Thereafter, a buffer solution is introduced into each column (Fig. 4A) so as to facilitate a polymerase reaction and a luciferase reaction as described below. The buffer solution used herein may comprise one such as buffer for sequencing, which includes: glycerol, glucose, APS, luciferin; TRIS-hydrochloric acid, etc., as a pH-buffering solution; $MgCl_2$ for forming a complex (e.g., a complex or $Mg^{2+}$ with dNTP or ATP) to be usable as a substrate for the polymerase reaction or luciferase reaction; NaCl, etc., as a salt solution for enhancing the reaction and for improving the stability; and dithiothreitol, etc., for protecting the enzymes protein against oxidative denaturation or deterioration.

(3) Into each of the columns in which the nucleic acid to be examined is hybridized with each of the primers contained therein, four kinds of dNTPs (i.e., dATP, dTTP, dGTP, and dCTP) are simultaneously added together with DNA polymerase as a catalyst (Fig. 4B). As a result, these deoxyribonucleotide units (monomers) including bases (A: adenine, T:thymine, G: guanine, C: cytosine) complementary to the respective bases constituting the nucleic acid to be examined are hybridized with the nucleic acid to be examined in

accordance with base pairing, whereby a unidirectional growth (or extension) reaction of each of the primers occurs in the direction corresponding to the direction of from 5' to 3' of the primer (as schematically shown in Fig. 4B).

Since the nucleic acid to be examined (for example, one having of a sequence of [AGCTGCTA]) is hybridized with the primers having sequences of [TCG], [CGA], [GAC], [ACG], and [GAT], respectively, 5 moles (n = 5, in the reaction formula (1) as described below) of pyrophosphoric acid (PPi) is released in the column to which the primer having a sequence of [TCG] has been fixed, in accordance with the following reaction formula (1) along with the growth reaction. Similarly, in the columns to which the primers having sequences of [CGA], [GAC], [ACG], and [GAT] have respectively been fixed, 2.5 moles, 3 moles, 2 moles, and 0 mole of PPi (n = 2.5, 3, 2, and 0, in the following reaction formula (1)) are released respectively.

template-primer + n $\times$ dNTP $\rightarrow$ template-(primer + n $\times$ dNMP) + n $\times$ PPi     Reaction Formula (1)

(4) The substrate or reagent for the emission reaction catalyzed by luciferase comprises luciferin, ATP, and the enzyme. However, in a case where dATP and APS are contained in the measurement system, they may also function as the substrate so as to somewhat cause emission reaction. Accordingly, in view of an improvement in the accuracy in the measurement, it is preferred to remove these substances by utilizing an enzymatic reaction before the emission reaction is conducted. More specifically, in such a case, hexokinase (such as a bead to which hexokinase has been fixed) may be added to each column, so that the excessive dATP, which has remained in the formation process represented by the above Reaction Formula (1), is caused to release Pi (phosphoric acid) therefrom thereby to form dADP (Fig. 4C).

When glycerokinase (such as a bead to which glycerokinase has been fixed) is added to each column, dATP which has remained in the column is caused to release Pi thereby to produce dADP in accordance with the following reaction formula (2).

dATP $\rightarrow$ dADP + Pi     Reaction Formula (2)

(5) Then, when ATP sulfurylase is added into each column, PPi remaining in the column is caused to react with APS so as to provide ATP in accordance with following reaction formula(3), as shown in Fig. 4D.

PPi + APS $\rightarrow$ ATP     Reaction Formula (3)

At this time, APS still remaining in each column even after the above reaction may be decomposed by adding AP sulfatase (adenosine 5'phosphosulfatase) into the column.

(6) Thereafter, the resultant capillary plate CA is placed in a dark box DB as shown in Fig. 5, which constitutes an apparatus for determining the base sequence of nucleic acid. The apparatus for determining the base sequence of a nucleic acid as shown in Fig. 5 comprises: a dark box DB for housing the capillary plate CA so as to shut off external light; a microscope or a macrolens 20 disposed on a surface on one side of the dark box DB, for observing the inside of each column formed in the capillary plate CA; a CCD television camera 10 for picking up an image of the capillary plate CA observed through the microscope or the macrolens 20; an image processing apparatus 30 for processing the image of a video signal supplied from the CCD camera 10 (for example, for filtering out a background optical signal having an intensity below a threshold value); and a computer 40 for determining the sequence of the nucleic acid to be examined by utilizing a single-stroke picturing algorithm, on the basis of the signal supplied from the image processing apparatus 30. The capillary plate CA is placed in the above apparatus for determining the base sequence of nucleic acid, and then luciferase (such as one in the form of a bead to which luciferase has been fixed) is added to each column (Fig. 4E), whereby light is emitted in a quantity which is proportional to the amount of the above ATP in accordance with the following reaction (luciferase reaction) formula (4), as shown in Fig. 4F.

luciferin + ATP + $O_2$ $\rightarrow$ oxyluciferin + AMP + PPi + $CO_2$ + light     Reaction Formula (4)

More specifically, the amount of ATP as described above is proportional to the amount of the PPi which has been released from the nucleic acid to be examined, and the amount of the PPi is proportional to the amount of bases which have been involved in the growth reaction. In addition, the primer is grown or extended only in the direction of from the 5'-end to the 3'-end, and therefore it is possible to determine the presence of the hybridization of the nucleic acid to be examined with the primer, and the position or the site of binding of the nucleic acid to be examined with the primer. When the resultant capillary plate CA is observed by using the apparatus for determining the base sequence of nucleic acid as shown in Fig. 5, a light emission phenomenon as shown in Fig. 6A may be observed. The arrangement of the primers in the columns of capillary plate CA is that as shown in Fig. 2 as described hereinabove.

More specifically, in the columns corresponding to the sequences of [TCG], [CGA], [GAC], [ACG] and [GAT], the emissions of light respectively having light quantities of 5, 2.5, 3, 2 and 0 are observed. When these sequences are arranged in an order of from the sequence corresponding to the smallest quantity of emission toward the sequence corresponding to a larger quantity of emission, by utilizing a single-stroke picturing algorithm, there may be determined the base sequence of TCGACGAT which has been formed on the basis of growth of the primer. Since the nucleic acid to be examined is a nucleic acid having the sequence complementary to such a sequence, the nucleic acid to be examined may be identified as [AGCTGCTA] (Fig. 6B).

As described above, based on measurement of the extended length of the primers after the growth thereof, mutual positional relationships among respective oligonucleotides which have been hybridized with the nucleic acid to be examined may be determined within the degree of accuracy or the precision of such measurement. When such measurement accuracy is improved so as to detect a difference corresponding to k-bases or smaller, as shown in Fig. 7, the base sequence of nucleic acid to be examined may be determined at this stage. Fig. 7 shows a method of hybridizing a nucleic acid to be examined having a base sequence of:

3'-GCCACCTCGAGGTTAAGCGGGATATCACTCAGCATAATCGCCGCGAGTGACC

GGCAGCAAAATGTT-5'

with primers of 8 mer disposed in respective columns; and shows a method of determining the base sequence of the nucleic acid to be examined by measuring the amount of pyrophosphoric acid released on the basis of a primer growth reaction. As described above, by use of the method according to this embodiment of the present invention, even when the nucleic acid to be examined has a longer chain, it is possible to determine the base sequence of the nucleic acid within a reasonably short period of time by using sets of longer primers to be disposed in the respective columns. In addition, when such a method for determining the base sequence is used, it is possible to determine or measure not only the kinds of the primers but also the positions of the binding thereof with the nucleic acid to be examined as a template. Accordingly, it is possible to determine the base sequence of nucleic acid to be examined to a considerable extent, even when the nucleic acid to be examined is one which can provide at least two Euler's ways or paths for single-stroke picturing so that the binding position therefor has not been identified.

In a case where the limit of error in the accuracy in the measurement exceeds k-bases, it is possible to determine the sequence of a portion by utilizing a computing algorithm described with respect to the above SBH method, even when the sequence of such a portion cannot be determined by the above method. In addition, in a case where precise and simple measurement is intended to be conducted, it is prererred to fix oligonucleotides (such as those of 8 mers) or the nucleic acid to be examined into a matrix-like member having a size such that it may be set in the measuring apparatus, or to form such oligonucleotides or nucleic acid into a compartment-like member, so that crossing, growth, and measurement (for example, those operations for 65536 combinations) may be conducted. Furthermore, when the nucleic acid or the oligonucleotide is fixed to the tube wall (or wall of the column), the operation for the growth reaction may be simplified.

Hybridization, release of pyrophosphoric acid associated with the hybridization, and light emission corresponding to the position of the binding of a primer with nucleic acid to be examined have been confirmed by using the following experiment wherein four types of dNTPs (i.e., dATP, dTTP, dGTP and dCTP) are actually introduced into respective columns together with DNA polymerase as a catalyst, and four types of dNTPs are added to the nucleic acid to be examined substantially simultaneously.

(Experiment Example 1)

In the above growth reaction utilizing DNA polymerase, when one dNTP molecule is incorporated into DNA, one pyrophosphoric acid molecule is released. Accordingly, the degree of promotion of the growth reaction may be determined by quantitatively measuring the amount of the pyrophosphoric acid which has been released on the basis of such reaction. An example of capillary plate to be used for an actual base sequencing apparatus (e.g., one having one million compartments) is equivalent or comparable to a bundle of one million test tubes. Accordingly, an experiment for experimentally demonstrating the above-described invention was conducted by using three test tubes, and the quantity of the resultant emission based on pyrophosphoric acid released from the nucleic acid to be examined was detected and measured. Here, fixation of the primers was not conducted, since it was independent of the phenomenon of light emission. In this experiment, pyrophosphoric acid was converted into ATP by using adenosine 5'-phosphosulfuric acid (APS) and ATP sulfurylase, and the resultant amount of ATP was detected on the basis of a light emission reaction utilizing luciferase.

(Materials and methods)

<Reagents>

Glycerokinase-fixed Sepharose beads
(i.e., Sepharose beads having glycerokinase fixed thereto)
Hexokinase-fixed Sepharose beads
ATP sulfurylase-fixed Sepharose beads
AP sulfatase-fixed Sepharose beads
Luciferase-fixed Sepharose beads
Single-stranded DNA having 224-base length,
(multicloning site of a bacterial plasmid)
Reverse primer
KS primer
DNA polymerase

dNTP buffer solution (dATP, dTTP, dGTP, dCTP: 25 μM each; glucose, glycerol: 10 mM each; APS: 1 μM, luciferin: 100 μg/ml, 40 mM-Tris·hydrochloric acid (pH 7.5), 20 mM-$MgCl_2$, 50 mM-NaCl, 10 mM-dithiothreitol)

The above reverse primer had a base sequence of [5'-AACAGCTATGACCATG-3'], and the KS primer had a base sequence of [5'-CGAGGTCGACGGTATCG-3'].

Further, a nucleic acid to be examined used herein had the following base sequence:

[3'-TTGTCGATACTGGTACTAATGCGGTTCGCGCG
TTAATTGGGAGTGATTTCCCTTGTTTTCGACCCATG
GCCCGGGGGGAGCTCCAGCTGCCATAGCTATTCGA
ACTATAGCTTAAGGACGTCGGGCCCCTAGGTGATC
AAGATCTCGCCGGCGGTGGCGCCACCTCGAGGTTAA
GCGGGATATCACTCAGCATAATGCGCGCGAGTGACC
GGCAGCAAAATG-5']

<Experimental Instruments>

A thermostatic oven
A photomultiplier-type integrating sphere equipped with a measurement holder for test tube
An universal photon counter(Hamamatsu Photonics K.K.)

<Experimental Operation>

First, three test tubes (a to c) were provided, and the following solutions were poured into the respective test tubes.

a) dNTP buffer solution (100 μl)
b) dNTP buffer solution (100 μl), single-stranded DNA (2 picomol), KS primer (1 picomol)
c) dNTP buffer solution (1 ml), single-stranded DNA (2 picomol), reverse primer (1 picomol)

Then, each of the above test tube was subjected to incubation at 65 °C for 3 minute, and immediately after the incubation, each test tube was cooled on ice. Thereafter, DNA polymerase (three units) was added thereto and each test tube was subjected to incubation at 37 °C for 5 minutes. Then, the test tubes were reserved on ice.

Suspensions of enzyme-fixed Sepharose beads were added each in an amount of 10 μl into each of the above test tubes in accordance with the following order (i.e., "1" to "4" as described below), and the resultant mixtures were gently stirred, and incubated at 25 °C for three minutes. Thereafter, each of the resultant supernatant liquids was transferred into a new test tube.

1. Glycerokinase-fixed Sepharose beads
2. Hexokinase-fixed Sepharose beads
3. ATP sulfurylase-fixed Sepharose beads
4. AP sulfatase-fixed Sepharose beads

10 μl of luciferase-fixed Sepharose beads (in this case, simple luciferase can be used) were added to each of the resultant final supernatant liquids and the resultant mixture was stirred gently, and immediately thereafter, each of the test tubes was set in the integrating sphere and the quantity of the resultant emission was counted. As a result of measurement of the emission, $0.19 \times 10^7$ photoelectron pulses were measured in a period of 204.8 seconds with respect to the test tube (a) containing no primer. Further, $2.06 \times 10^7$ photoelectron pulses were measured with respect to the test tube (b) containing the reverse primer, and $1.45 \times 10^7$ photoelectron pulses were measured with respect to the test tube (c) containing the KS primer.

In other words, it was expected that the lengths of the growth based on the reverse primer and the KS primer by hybridization with the above nucleic acid to be examined were 208 and 127, respectively, on the basis of the base sequence of the nucleic acid to be examined. Thus, the amounts of the pyrophosphoric acid released along with the hybridization were expected to be 208 and 127, respectively. Accordingly, the above experimental results show that the lengths of the respective grown primers may be estimated within an error range of about 13-bases. The error of the about 13-bases is considered to be that due to background light emission. Therefore, it is possible to improve the detection accuracy by improving the measuring apparatus such that the background light emission may be suppressed. In other words, when the above method for determining the base sequence is used, it is possible to determine or measure not only the kinds of the binding primers but the positions of the binding thereof with the nucleic acid to be examined as a template, for short period of time. Accordingly, it is possible to determine the base sequence of a nucleic acid to be examined to a considerable extent by using the above method for determining the base sequence of nucleic acid, even when the nucleic acid to be examined is one which can provide at least two Euler's paths for single-stroke picturing so that the binding position therefor has not been identified.

Second Embodiment

In above First Embodiment, the base sequence of a nucleic acid to be examined was determined by hybridizing the primers with the nucleic acid to be examined and by quantitatively measuring the amounts of pyrophosphoric acid released by the hybridization. Next, there is described a method of determining the base sequence of nucleic acid by use or fluorescence-labelled dNTPs (or dNTPs labelled with a fluorescent substance).

(1) In the same manner as in First Embodiment, a capillary plate (CA) in a square shape having plural columns formed therein as shown in Fig. 1 is provided, and a plurality of oligonucleotides including all of the combinations comprising k bases as plural primers are fixed into the above respective columns. More specifically, trimers (3 mers) of all of the combinations containing A, T, G, and C, are fixed into 64 columns of the glass capillary plate, as shown in Fig. 8.

(2) Then, into one of the columns in the capillary plate CA having a primer fixed thereto (for example, into the column having a primer with a sequence of [GAC], unknown single-stranded nucleic acid to be examined (for example, one having a base sequence of [AGCTGCTA]) is introduced (Fig. 8). Based on such introduction, the primer disposed in each column is hybridized (or crossed) with the nucleic acid to be examined. Then, the nucleic acid to be examined which has not been hybridized with the primer is washed out. Thereafter, a buffer solution is introduced into each column (Fig. 9A) so as to facilitate a polymerase reaction and a luciferase reaction as described below. The buffer solution used herein is the same as that used in First Embodiment.

(3) Into each of the columns in which the nucleic acid to be examined has been hybridized with each of the primers contained therein, four kinds of fluorescent-dNTPs (i.e., dATP, dTTP, dGTP, and dCTP) are simultaneously added together with DNA polymerase as a catalyst (Fig. 9B). As a result, these deoxyribonucleotide units (monomers) including bases (A: adenine, T:thymine, G: guanine, C: cytosine) complementary to the respective bases constituting the nucleic acid to be examined are hybridized with the nucleic acid to be examined in accordance with base pairing, whereby a unidirectional growth (or extension) reaction of each of the primers occurs in the direction corresponding to the direction of from 5' to 3' of the primer (as schematically shown in Fig. 9B). Thereafter, the fluorescent-dNTP is removed from the inside of the column by washing.

Since the nucleic acid to be examined (for example, one having of the sequence of [AGCTGCTA]) is hybridized with the primers having sequences of [TCG], [CGA], [GAC], [ACG], and [GAT], respectively, 5 moles (n = 5, in the reaction formula (5) as described below) of fluorescent dye or fluorochrome is incorporated into the column to which the primer having a sequence of [TGC] has been fixed, in accordance with the following reaction formula (5) along with the growth reaction. Similarly, in the columns to which the primers having sequences of [CGA], [GAC], [ACG], and [GAT] have respectively been fixed, 2.5 moles, 3 moles, 2 moles, and 0 mole of the fluorescent dye (n = 2.5, 3, 2, and 0, in the following reaction formula (5)) are respectively incorporated.

template-primer + n $\times$ fluorescent-dNTP $\rightarrow$ template-(primer + n $\times$ fluorescent-dNMP) + n $\times$ PPi Reaction Formula (5)

(4) Thereafter, the resultant capillary plate CA is placed in a dark box DB as shown in Fig. 10A, which constitutes an apparatus for determining the base sequence of nucleic acid. The apparatus for determining the base sequence of nucleic acid as shown in Fig. 10A comprises: the dark box DB1 for housing the capillary plate CA so as to shut off external light; an excitation light source 70 (such as Xenon lamp) for supplying excitation light to the capillary plate CA; and a CCD television camera 10 disposed on a surface on one side of the dark box DB1, for picking up an image of the capillary plate CA.

Further, an excitation light-transmitting filter 50 is disposed between the excitation light source 70 and the capillary plate CA so that among the light component having various wavelengths, one having a shorter wavelength (for example, one having a peak wavelength of 450 nm) selectively transmits the filter as shown in Fig. 10C to irradiate the capillary plate CA with the thus transmitted light. In addition, a fluorescence-transmitting filter 60 is also disposed between the capillary plate CA and the CCD television camera 10, so that light having a wavelength longer than the fluorescence wavelength (for example, wavelength of 500 nm or longer) is selectively transmitted the filter as shown in Fig. 10B. As the excitation light source 70, an ultra-high pressure mercury lamp 701 as shown in Fig. 10D may also be employed.

(5) After the capillary plate CA is placed in such an apparatus for determining the base sequence of nucleic acid, excitation light (for example, light having a wavelength of 450 nm) emitted from the excitation light source 70 is supplied onto the capillary plate CA, and an image of fluorescence is observed. The resultant image of fluorescence obtained by such observation is the same as the above-mentioned image shown in Fig. 6A, and the sequence of the nucleic acid to be examined may be determined in the same manner as described in First Embodiment. More specifically, the amount of growth of each primer is proportional to the amount of fluorescence emitted from the fluorescent dye involved in the above growth reaction. Since the primer used herein grows or extends only in the direction of from the 5'-end to the 3'-end thereof, both of the presence of the hybridization of the nucleic acid to be examined with the primer, and the position of binding of the nucleic acid to be examined with the primer may be determined. The arrangement of the primers in the columns of the capillary plate CA is the same as that shown in Fig. 2, as described hereinabove.

More specifically, in the columns corresponding to the primer sequences of [TCG], [CGA], [GAC], [ACG] and [GAT], the emissions of light respectively having light quantities of 5, 2.5, 3, 2 and 0 are observed. When these sequences are arranged in an order of from the sequence corresponding to the smallest quantity of emission toward the sequence corresponding to a larger quantity of emission, by utilizing a single-stroke picturing algorithm, there may be determined a base sequence of TCGACGAT which has been formed on the basis of the growth of the primer. Since the nucleic acid to be examined is nucleic acid having the sequence complementary to such a sequence, the nucleic acid to be examined may be identified as [AGCTGCTA] (Fig. 6B). Such a method may also determine the base sequence of long-chain nucleic acid as shown in Fig. 7.

Hybridization, and light (fluorescence) emission which is proportional to the position of the binding of a primer with nucleic acid to be examined have been confirmed by using the following experiment wherein one of the four types of fluorescent dye-dNTPs (i.e., fluorescent dye-dATP, fluorescent dye-dTTP, fluorescent dye-dGTP and fluorescent dye-dCTP) was actually introduced into respective columns together with a DNA polymerase as a catalyst, and the four types of dNTPs are added to the nucleic acid to be examined substantially simultaneously.

(Experiment Example 2)

The amount of dNTPs to be incorporated into the nucleic acid along with the growth reaction was quantitatively measured. When dNTPs were incorporated into DNA in the growth reaction catalyzed by DNA polymerase, at least one kind of fluorescent dye-dNTP was used instead of a part of or all of the dNTPs to be used for such a purpose. When fluorescein-12-dUTP (or rhodamine-4-dUTP) compatible with dTTP was partially used, growth or extention reaction occurred. In this case, the nucleic acid hybridized with the dNTP should be fixed on a wall of the column, etc., in order to remove the unreacted fluorescence-labelled dNTPs by washing. In this Example, as an alternative operation, the washing was conducted after the fixing thereof onto magnetic beads.

(Materials and Method)

<Reagents>

Streptoavidin-fixed magnetic beads (Dynabeads M-280, mfd. by DYNAL Co.)

Single-stranded DNA having a 224-base length, the 3'-end of which is biotinylated (multicloning site of plasmid)

Reverse primer

KS primer

DNA polymerase (trade name: AmpliTaq™ DNA Polymerase, mfd. by PERKIN-ELMER Co.)

dNTP buffer solution (dATP, dGTP, dCTP: 25 μM each; dTTP: 8 μM, fluorescein-12-dUTP (or rhodamine-4-dUTP): 2 μM, 10 mM-Tris-hydrochloric acid (pH 8.3), 1.5 mM-$MgCl_2$, 50 mM-KCl, 0.01 % gelatin)

Washing solution (10 mM-Tris-hydrochloric acid (pH 7.5), 2 mM-EDTA, 1 M-NaCl)

80 % formamide aqueous solution

<Experimental Instruments>

A test tube holder equipped with magnet

A thermostat oven for PCR (polymerase chain reaction)

A fluorescence spectrophotometer

<Experimental Operations>

First, two test tubes (a and b) were provided, and the following solutions were poured into the respective test tubes.

a) dNTP buffer solution (50 μl), single-stranded DNA (1 picomol), DNA polymerase (1 unit), reverse primer (2 picomol)

b) dNTP buffer solution (50 μl), single-stranded DNA (1 picomol), DNA polymerase (1 unit), KS primer (2 picomol)

Then, each of the above test tubes was subjected to incubation at 95 °C (30 seconds), at 41 °C (30 seconds), and at 72 °C (5 minutes), in this order. Immediately after the incubation, each test tube was maintained at 4 °C.

Thereafter, magnetic beads (50 μl) were added to the resultant mixture and subjected to stirring, and each of the test tubes was set in the holder equipped with a magnet. The supernatant liquid was removed by using a pipet so that the pipet did not contact the magnetic beads. The magnetic beads were then washed with a washing solution twice, and washed with distilled water once. Then, the resultant magnetic beads were suspended in 500 μl of 80 % formamide aqueous solution, and subjected to incubation at 95 °C for three minutes, thereby to elute a grown complementary strand.

Then, each tube was again set in the holder equipped with a magnet, and the resultant supernatant liquid containing the grown complementary strand was taken out and transferred into a cuvette for a spectrophotometer, and a fluorescence spectrum was measured. The excitation light used herein was that having a wavelength of 488 nm in the case where the fluorescent dye comprised fluorescein, or that having a wavelength of 540 nm in the case where the fluorescent dye comprised rhodamine. Fig. 11 shows fluorescence spectra of DNA which has incorporated the fluorescein(A) or rhodamine(B). Further, for the purpose of measuring the length of the growth by using the fluorescence dye, the intensities of the fluorescence at 520 nm and 580 nm were used. As a result of measurement of these fluorescence intensities (quantity of the emission), when the fluorescein was used as the fluorescence dye, a fluorescence intensity of 3.35 (a.u.; arbitrary unit) was observed with respect to the test tube (a) containing the reverse primer, and a fluorescence intensity of 1.71 (a.u.) was observed with respect to the test tube (b) containing the KS primer. Furthermore, when the rhodamine was used as the fluorescence dye, a fluorescence intensity of 10.10 (a.u.) was observed with respect to the test tube (a) containing the reverse primer, and a fluorescence intensity of 5.26 (a.u.) was observed with respect to the test tube (b) containing the KS primer.

The ratio between the above fluorescence intensities with respect to the two growth reactions (i.e., the growth reactions in the test tubes (a) and (b)) was about 1.9, when either of the fluorescent dye was used for such a purpose. The ratio of the lengths (numbers of the bases) in the actual growth reaction was 208 / 127 = 1.63. However, when the ratio between numbers of the fluorescence dye (dUTP) to be incorporated into the nucleic acid (i.e., the ratio between the numbers of the positions corresponding to "T" ) was calculated, the ratio was found to be 1.96. It was found that this ratio (1.96) was very close to the actually measured values

(about 1.9) with respect to the fluorescence intensities.

As described above, the ratio of the lengths of growth based on the reverse primer and the KS primer caused by the hybridization thereof with the above nucleic acid to be examined may be determined by the above fluorescent intensities to a considerable extent, even when one kind of the fluorescence dye (dNTP) is used. In addition, there may be recognized both of the positions of the binding between the primers and nucleic acid to be examined, and the kinds of the primers bound to the nucleic acid. Accordingly, in this embodiment, the base sequence of the nucleic acid to be examined was also determined by using the single-stroke picturing algorithm in the same manner as in First Embodiment. Further, not only the kinds of primers but also the positions of the binding of the primers with the nucleic acid to be examined as a template may be precisely measured by improving the measurement accuracy. Accordingly, it is possible to determine the base sequence of nucleic acid having at least two Euler's paths for single-stroke picturing, even when the nucleic acid has a base sequence which has not been determined in the prior art because of the impossibility of binding position identification.

The present invention is not limited to the above embodiments. More specifically, in the above First and Second Embodiments, the length of the growth is determined (i) by measuring the amount of pyrophosphoric acid released along with the growth reaction; or (ii) by measuring the emission from the fluorescent label. In addition, the following methods may also be used as the method of determining the length of the growth.

(I) A method of measuring the amount of a decomposition product which is released along with the growth reaction.

(II) A method of measuring the amount of bases which are incorporated into nucleic acid in the growth reaction.

(III) A method of directly observing the length of the resultant double-stranded portion of nucleic acid to be examined after the growth reaction, by use of an electron microscope or a scanning tunnelling microscope having a resolution corresponding to the atomic level.

(IV) A method of separating the resultant product per se of the growth reaction (nucleic acid comprising complementary strands), and subjecting the separated product to gel electrophoresis thereby to determine the length thereof in the same manner as in a length-determining method which has actually been used at present.

In the above First and Second Embodiments, a capillary plate CA having a square shape is used, since desired primers may easily be disposed in a matrix-like shape. However, in the present invention, a capillary plate having a circular shape may also be used instead of such a square plate.

As described hereinabove, when the method of determining the base sequence of nucleic acid according to the present invention is used, in a first step, a primer and a nucleic acid to be examined are subjected to hybridization to form a complex therebetween; in a second step, growth reaction in the direction of from 5' to 3' of the primer is conducted by using the nucleic acid to be examined as a template; and in a third step, the amount of the growth of the primer is detected. Through such a procedure, each of the amounts of growth of each of the primers disposed in plural regions, i.e., the position of the binding of the primer with the nucleic acid to be examined and the kind of each of the bound primers in each region, may be identified within a short period of time. Accordingly, the base sequence of nucleic acid to be examined may be determined by analyzing the base sequences of the primers by using a so-called single-stroke picturing algorithm.

Further, when the measuring accuracy is further improved, not only the kinds of the primers but also the positions of the binding of the primers with the nucleic acid as a template, may be precisely measured. Accordingly, the base sequence of a nucleic acid having at least two Euler's paths for single-stroke picturing may also be identified, even when the base sequence of such a nucleic acid cannot be determined because of impossibility of binding position identification. Furthermore, when the above-mentioned plural regions are defined by columns in capillary plate (e.g., one comprising a glass material or a silicon material), the mixing of the primers with each other may be prevented.

As the above method of detecting the amount of the growth, there may be used a method of detecting the amount of the light emission provided by the luciferase reaction with ATP converted from pyrophosphoric acid which has been released from plural kinds of nucleotides or nucleotide analogues in the growth reaction; or a method wherein primers is subjected to a growth reaction together with the addition of fluorescence-labelled deoxyribonucleoride triphosphates (dNTPs) as nucleotide analogues in the presence of the nucleic acid to be examined as a template, and the quantity of the fluorescence emission based on the fluorescent label is detected. Through such a procedure, the amount of emission is proportional to the amount of the growth, and therefore the kinds of the primers bound to the nucleic acid to be examined and the positions of the binding of the primers with the nucleic acid to be examined may be determined.

From the invention thus described, it will be obvious that the invention may be varied in many ways. Such variations are not to be regarded as a departure from the spirit and scope of the invention, and all such modifications as would be obvious to one skilled in the art are intended to be included within the scope of the following claims.

The basic Japanese Application No.6-006971/1994 filed on January 26, 1994 is hereby incorporated by reference.

Further, papers relating to the above-mentioned SBH and SHOM methods (Bains, W and Smith, G. C., J. Theor. Biol. 135: 303-307, 1988; Darmance, R., Labat, I., Brukner, I. and Crkvenjakov, R., Genomics 4: 114-128, 1989 / Pevzner, P. A., J. Biomol. Struct. Dyn. 7: 63-73, 1989 / Pevzner, P. A., Lysov, Y. P., Khrapko, K. R. Belyavskii, A. V., Florent ev, V. L. and Mirzabekov, A., J. Biomol. Struct. Dyn. 9: 399-415, 1991) are hereby incorporated by reference.

## Claims

1. A method of determining the base sequence of single-stranded nucleic acid, comprising:

   a first step of hybridizing each of the plural primers comprising an oligonucleotide, with a template comprising single-stranded nucleic acid to be examined, thereby to form a complex having a double-stranded portion comprising a portion of the template and the primer; the plural primers being separately disposed in a plurality of regions in accordance with the kinds of the primers;

   a second step of substantially simultaneously adding the plural kinds of nucleotides or nucleotide analogues to the complexes respectively disposed in the plural regions, thereby to perform growth (or extention) reaction of the primer with the nucleic acid to be examined as the template in a direction of from 5' to 3' of the primer; the plural kinds of nucleotides or nucleotide analogues being complementary to nucleotide constituting the template in the complex, and being capable of forming base pair with nucleotide constituting the template; and

   a third step of detecting the amount of the growth of the primers.

2. A method of determining the sequence of nucleic acid according to Claim 1, wherein the plural regions are provided by respective columns formed in a capillary plate.

3. A method of determining the sequence of nucleic acid according to Claim 2, wherein the capillary plate comprises a glass material or a silicon material.

4. A method of determining the sequence of nucleic acid according to Claim 1, wherein in the third step, the amount of the growth of the primer is determined by causing APS (adenosine 5'-phosphosulfate) to react with pyrophosphoric acid which has been released from the plural kinds of nucleotides or nucleotide analogues along with the growth reaction in the second step, thereby to produce ATP (adenosine triphosphate); and detecting the quantity of light emitted on the basis of the luciferase reaction of the resultant ATP.

5. A method of determining the base sequence of nucleic acid according to Claim 1, wherein a deoxyribonucleotide triphosphate (dNTP) labelled with a fluorescent substance as at least one of the plural kinds of nucleotide analogues in the second step is added so as to perform the growth reaction of the primer with the nucleic acid to be examined as the template, and

   the plural regions are irradiated with excitation light and the quantity of light emitted from the fluorescent label of the grown primers is detected, thereby to determine the amount of the growth of the primers.

6. A method for use in determining the base sequence of a single stranded nucleic acid comprising:

   i) hybridizing each of a plurality of oligonucleotide primers to separate samples of single stranded nucleic acid

   ii) extending each oligonucleotide to elongate the double stranded region of each sample

   iii) quantitating the extension of the double stranded region of each sample.

7. A method according to claim 6 wherein the number of primers corresponds to all possible base combinations of the chosen primer length.

Fig. 1

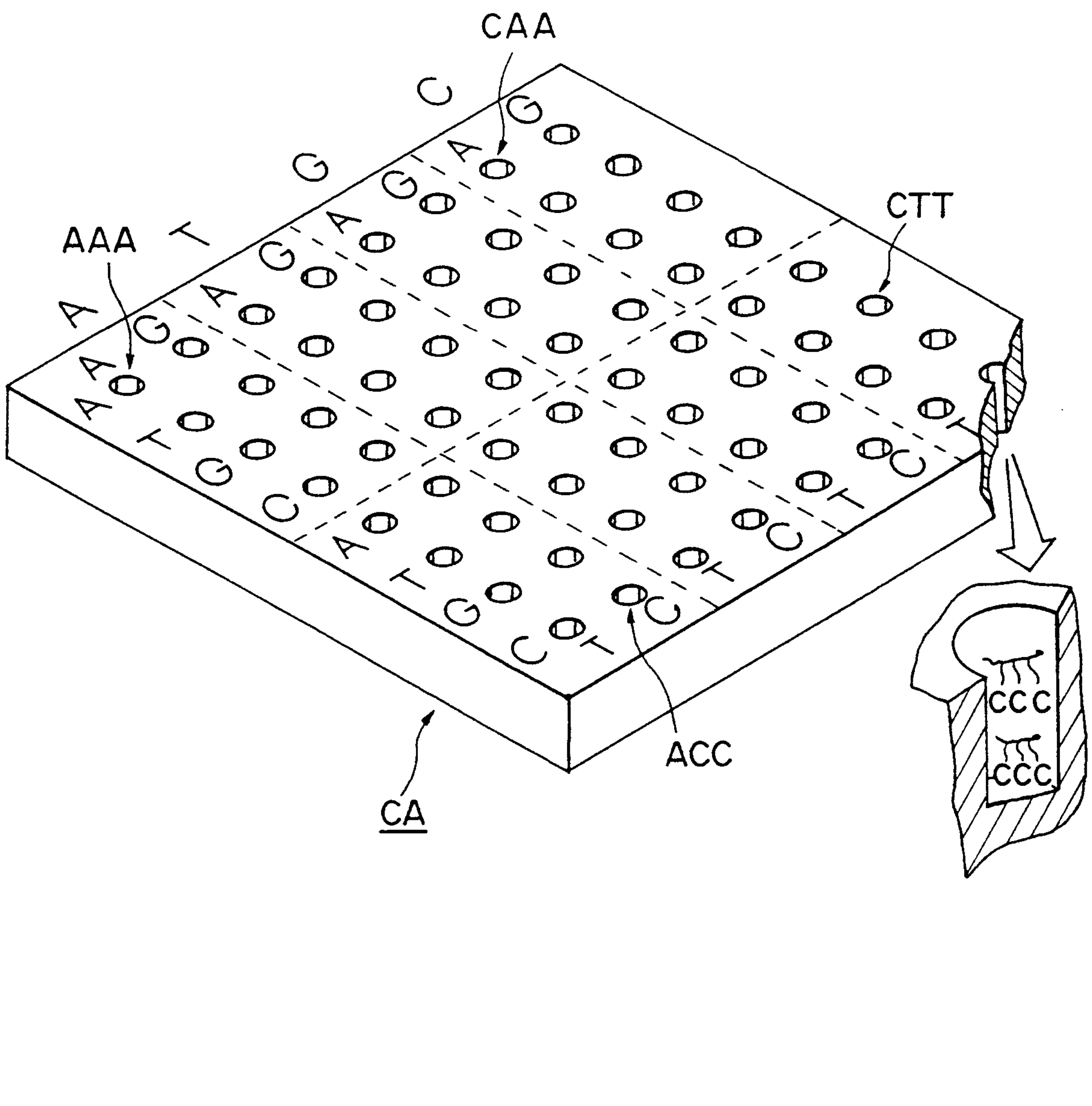

Fig. 2

| j s f | j s f | j s f | j s f | j s f | j s f | j s f | j s f |
|---|---|---|---|---|---|---|---|
| AAA | AGA | TAA | TGA | GAA | GGA | CAA | CGA |
| AAT | AGT | TAT | TGT | GAT | GGT | CAT | CGT |
| AAG | AGG | TAG | TGG | GAG | GGG | CAG | CGG |
| AAC | AGC | TAC | TGC | GAC | GGC | CAC | CGC |
| ATA | ACA | TTA | TCA | GTA | GCA | CTA | CCA |
| ATT | ACT | TTT | TCT | GTT | GCT | CTT | CCT |
| ATG | ACG | TTG | TCG | GTG | GCG | CTG | CCG |
| ATC | ACC | TTC | TCC | GTC | GCC | CTC | CCC |

ARRANGEMENT OF PRIMERS IN CAPILLARY PLATE

Fig. 3

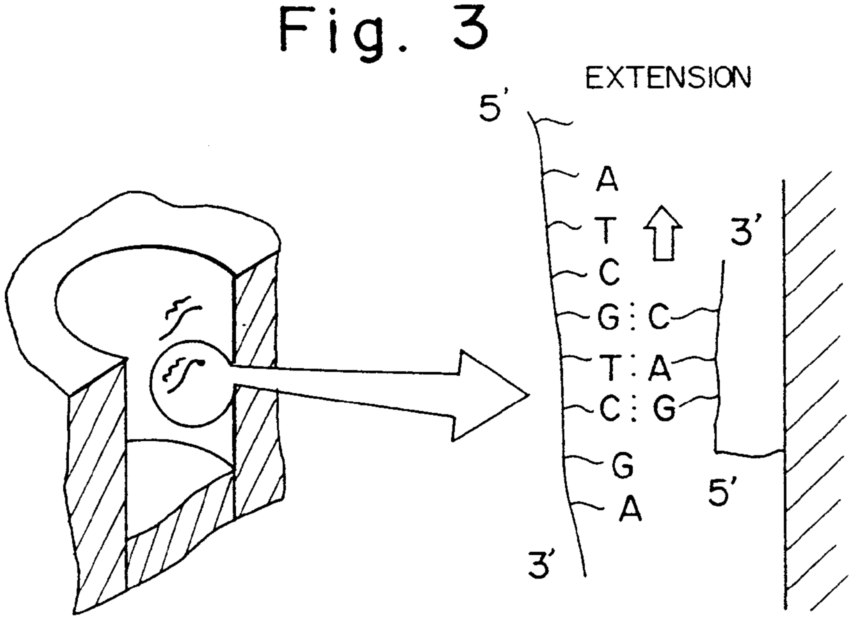


Fig. 4A

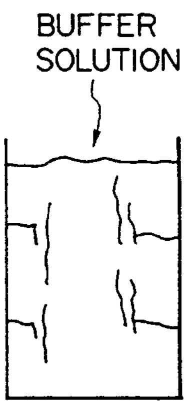


(INITIAL STATE)

Fig. 4B

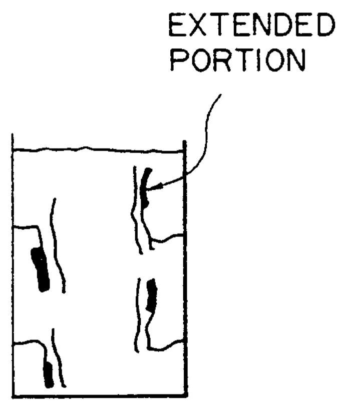


(EXTENSION REACTION) PPi RELEASE

Fig. 4C

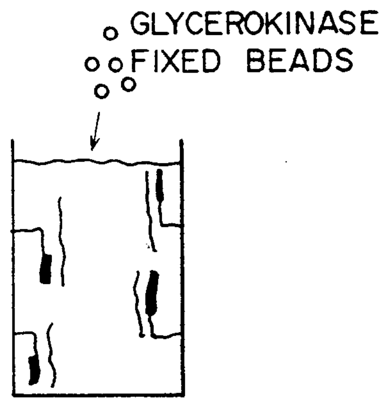


(REMOVAL OF ATP)

Fig. 4D

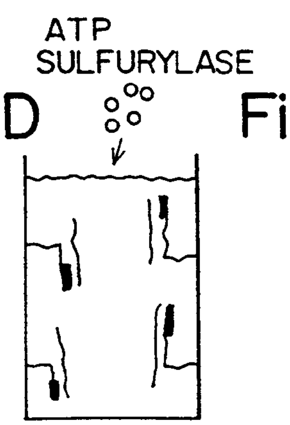


(DECOMPOSITION OF RESIDUAL APS)

Fig. 4E

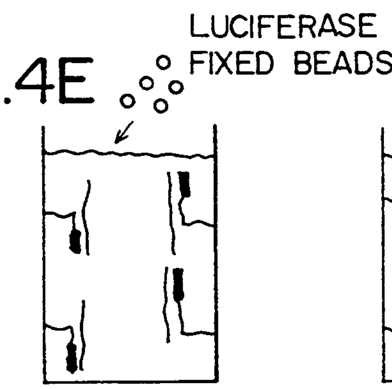


Fig. 4F

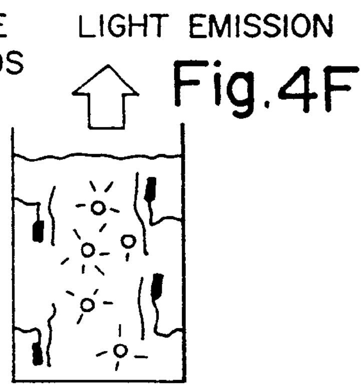


(STEP OF OBSERVING OF EMISSION)

Fig. 5

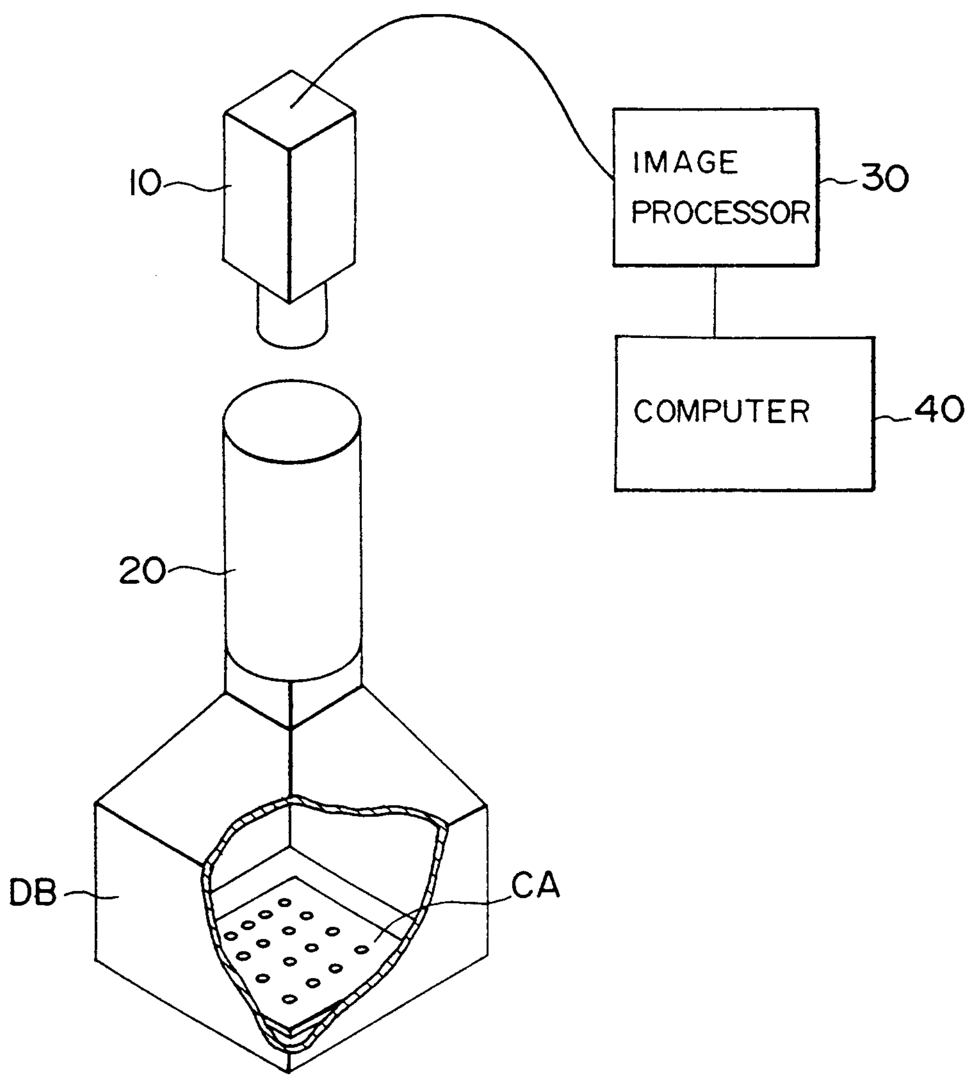

Fig. 6A

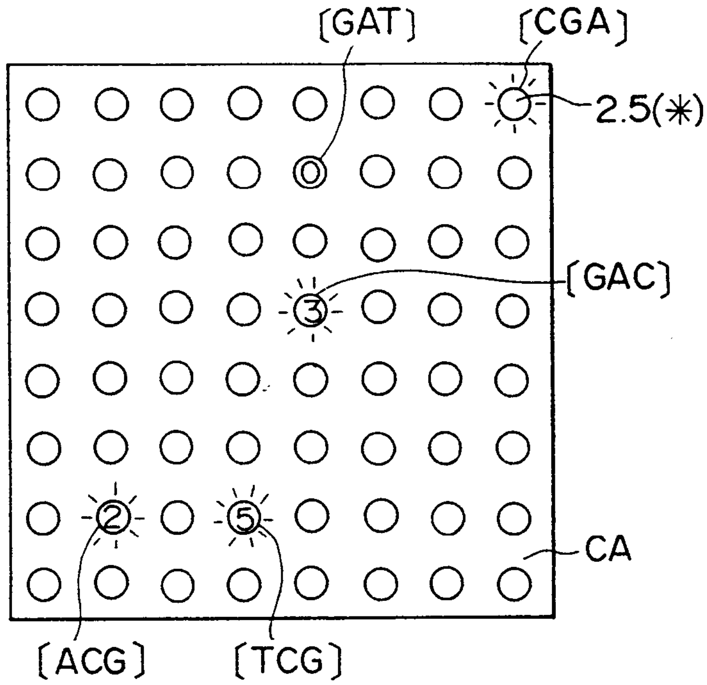


NUCLEIC ACID TO BE EXAMINED
AGCTGCTA

Fig. 6B

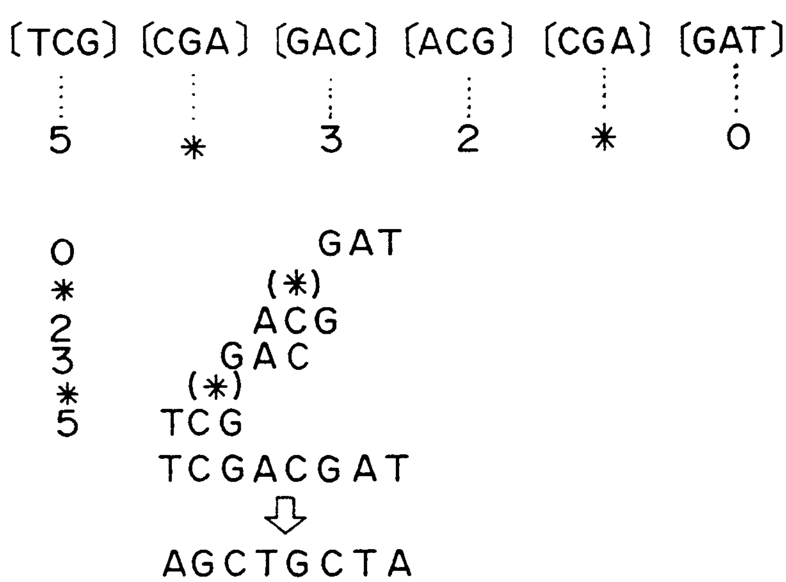

Fig. 7

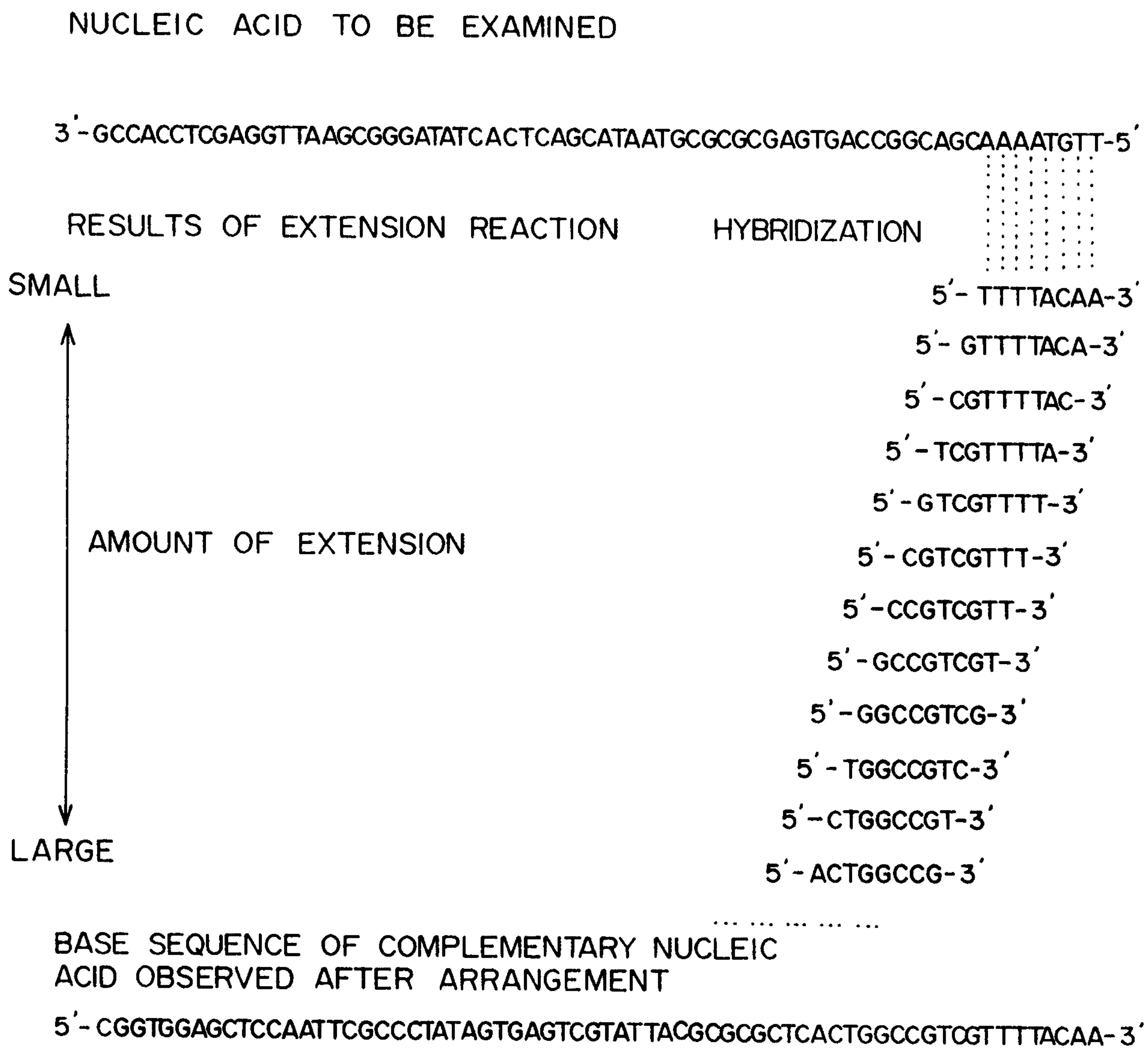
NUCLEIC ACID TO BE EXAMINED
3'-GCCACCTCGAGGTTAAGCGGGATATCACTCAGCATAATGCGCGCGAGTGACCGGCAGCAAAATGTT-5'
RESULTS OF EXTENSION REACTION
HYBRIDIZATION
SMALL
AMOUNT OF EXTENSION
LARGE
5'- TTTTACAA-3'
5'- GTTTTACA-3'
5'-CGTTTTAC-3'
5'-TCGTTTTA-3'
5'-GTCGTTTT-3'
5'-CGTCGTTT-3'
5'-CCGTCGTT-3'
5'-GCCGTCGT-3'
5'-GGCCGTCG-3'
5'-TGGCCGTC-3'
5'-CTGGCCGT-3'
5'-ACTGGCCG-3'
... ... ... ... ...
BASE SEQUENCE OF COMPLEMENTARY NUCLEIC ACID OBSERVED AFTER ARRANGEMENT
5'-CGGTGGAGCTCCAATTCGCCCTATAGTGAGTCGTATTACGCGCGCTCACTGGCCGTCGTTTTACAA-3'

Fig. 8

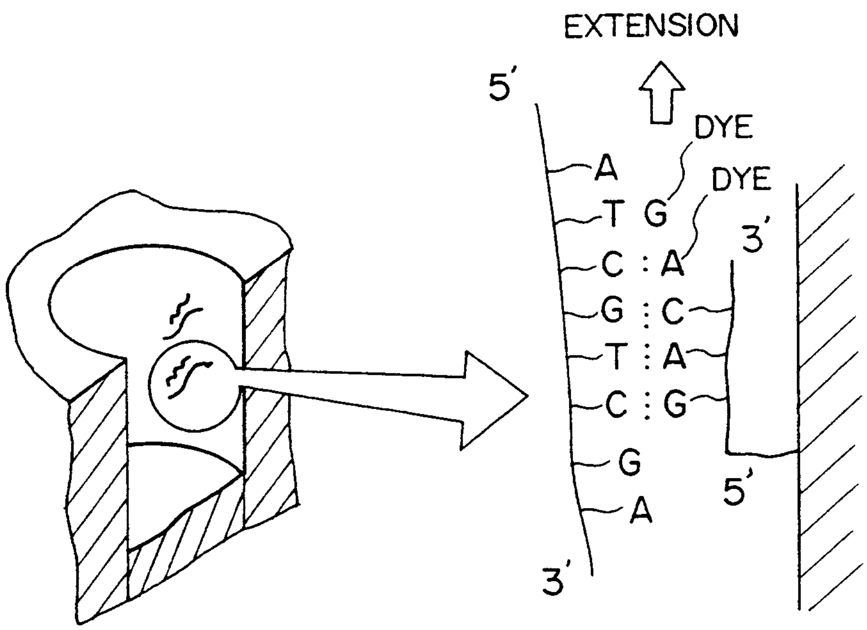


Fig. 9A

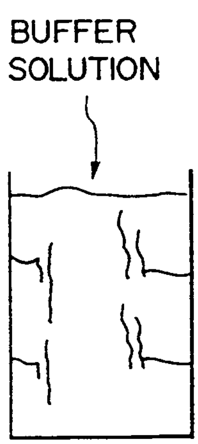


(INITIAL STATE)

Fig. 9B

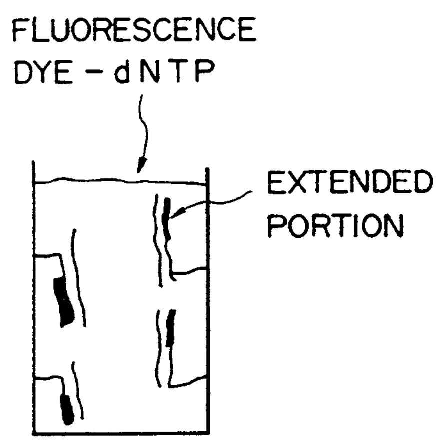


(EXTENSION REACTION)
PPi RELEASE

Fig.10A

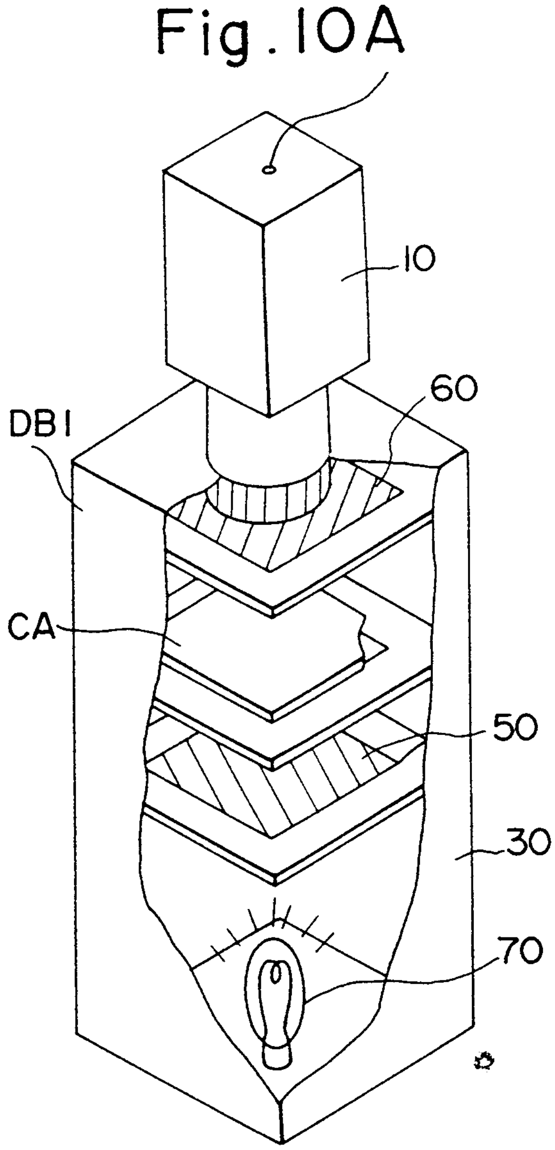


Fig.10B

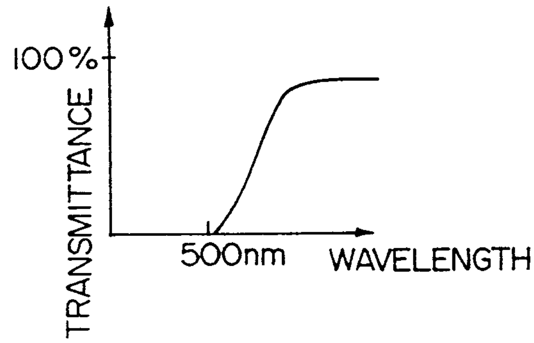


Fig.10C

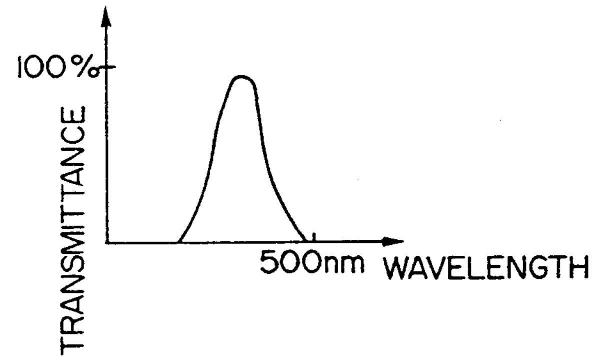


Fig.10D

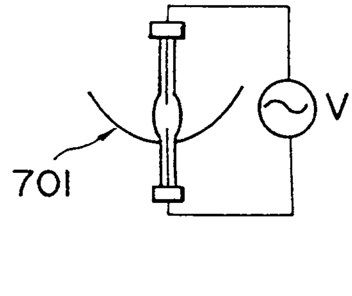

Fig. 11

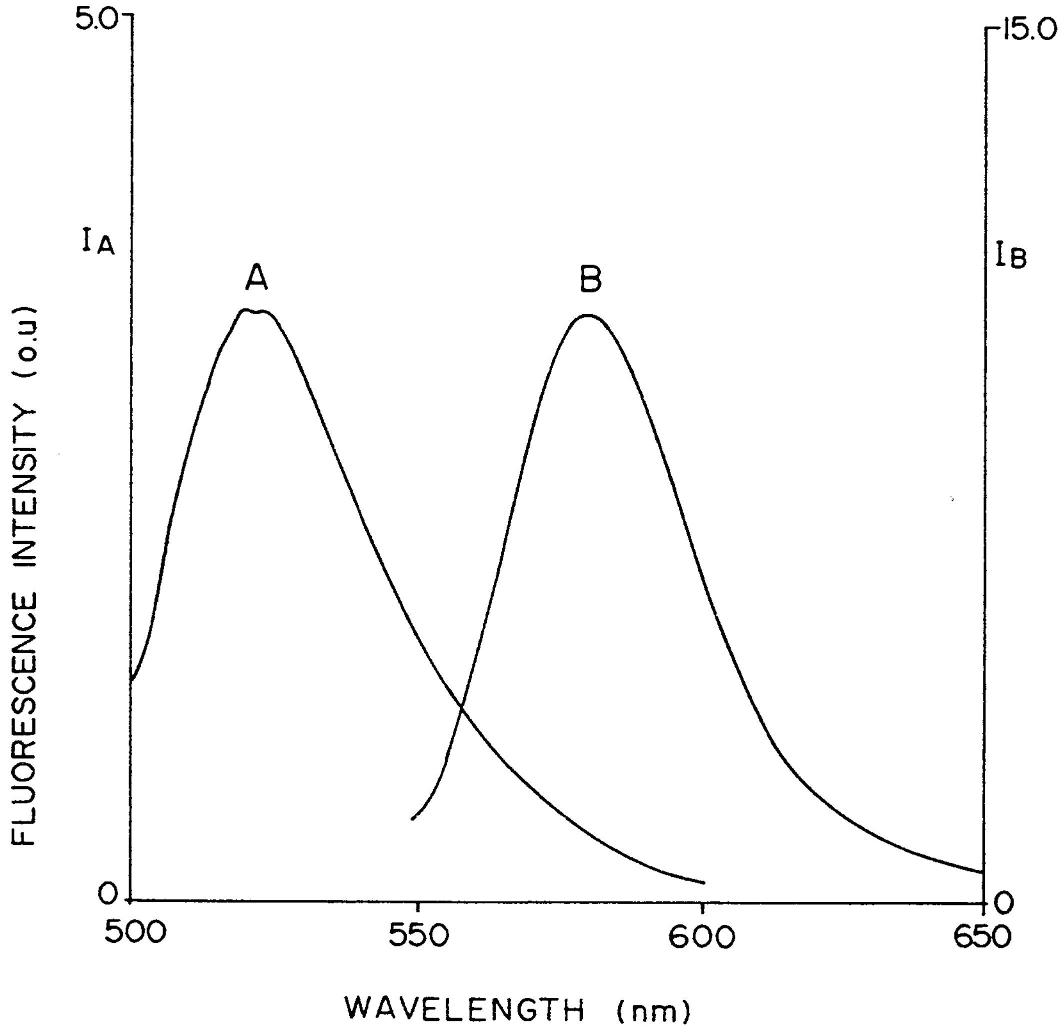
5.0
15.0
IA
IB
A
B
FLUORESCENCE INTENSITY (o.u)
0
0
500
550
600
650
WAVELENGTH (nm)

Fig. 12A

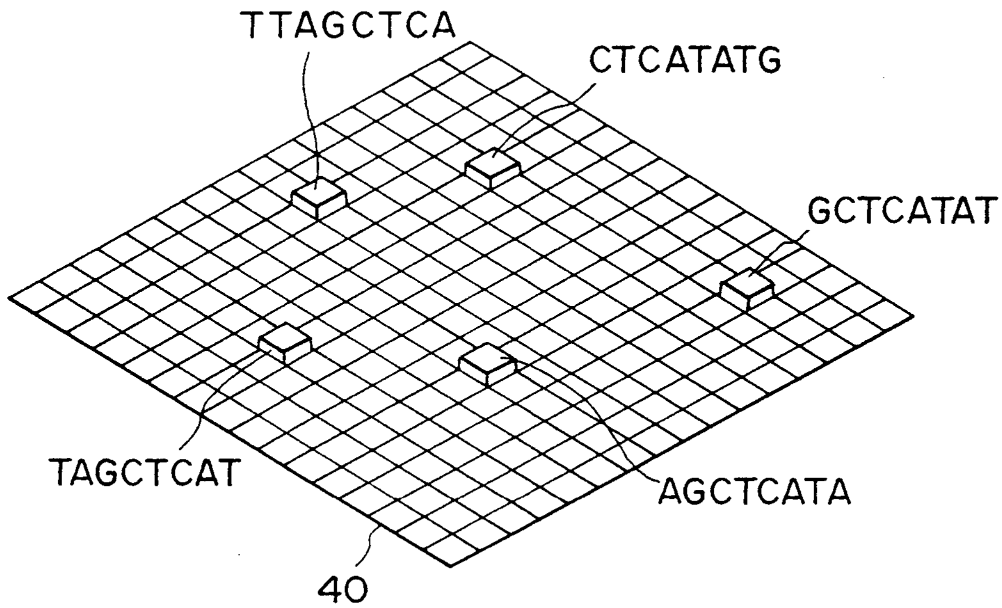


Fig. 12B

```
                                   TTAGCTCA
                                    TAGCTCAT
                                     AGCTCATA
                                      GCTCATAT
                                       CTCATATG
                                   ____________
                                   TTAGCTCATATG
(NUCLEIC ACID TO BE EXAMINED) -->  AATCGAGTAGAC
```